(19) Europäisches Patentamt
European Patent Office
Office européen des brevets

(11) EP 3 940 056 A1

(12) **EUROPEAN PATENT APPLICATION**
published in accordance with Art. 153(4) EPC

(43) Date of publication:
**19.01.2022 Bulletin 2022/03**

(21) Application number: **19899645.6**

(22) Date of filing: **12.12.2019**

(51) International Patent Classification (IPC):
***C12N 1/21** (2006.01)* ***C12N 15/31** (2006.01)*
***C07K 14/36** (2006.01)* ***C12P 19/62** (2006.01)*
***C12R 1/465** (2006.01)*

(52) Cooperative Patent Classification (CPC):
**C07K 14/36; C12N 1/205; C12P 19/62;**
C12R 2001/465

(86) International application number:
**PCT/CN2019/124724**

(87) International publication number:
**WO 2020/125531 (25.06.2020 Gazette 2020/26)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **20.12.2018 CN 201811577592**

(71) Applicant: **Shenyang Fuyang Pharmaceutical Technology Co., Ltd.**
**Shenyang, Liaoning 110164 (CN)**

(72) Inventors:
- **JIANG, Enhong**
**Shenyang, Liaoning 110164 (CN)**
- **HE, Weiqing**
**Shenyang, Liaoning 110164 (CN)**
- **ZHAO, Xiaofeng**
**Shenyang, Liaoning 110164 (CN)**
- **JIANG, Xunlei**
**Shenyang, Liaoning 110164 (CN)**

(74) Representative: **Murgitroyd & Company**
**Murgitroyd House**
**165-169 Scotland Street**
**Glasgow G5 8PL (GB)**

# (54) SPIRAMYCIN-PRODUCING STRAIN, KELIMYCIN-PRODUCING STRAIN, CONSTRUCTION METHOD THEREFOR, USE THEREOF AND METHOD FOR INCREASING PRODUCT YIELD THEREOF

(57) The present disclosure provides a spiramycin-producing strain, a carrimycin-producing strain, construction method therefor, use thereof and method for increasing the product yield thereof. The provided spiramycin-producing strain has an inactivated gene Lrp (ΔIrp-SP); and the strain has a preservation number of CGMCC No. 16056. The provided carrimycin-producing strain has an inactivated gene Lrp (ΔIrp-BT); and the strain has a preservation number of CGMCC No.16055. By inactivating the gene Lrp, the yields of spiramycin and carrimycin are increased; and particularly the yield and proportion of a major component of the carrimycin, that is, 4"-O-isovalerylspiramycin III are significantly increased.

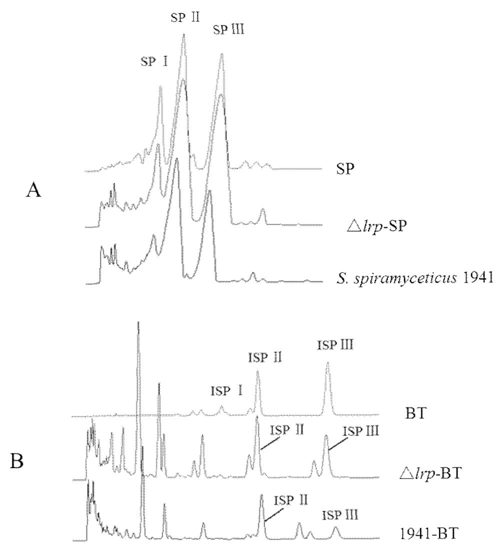


Fig. 5

EP 3 940 056 A1

## Description

### TECHNICAL FIELD

The present disclosure belongs to the field of microbial genetic engineering, relates to a spiramycin-producing strain, a carrimycin-producing strain, construction method therefor, use thereof and method for increasing product yield, and particularly relates to a method for the operation of regulatory genes and increasing the antibiotic yield and effective components and application thereof.

### BACKGROUND

Carrimycin has former names such as Shengjimycin or Bitespiramycin, and is a fermentation product [Patent No.: ZL971044406, ZL021487715] of a genetically engineered bacterium *(Streptomyces spiramyceticus* WSJ-1) obtained by cloning and expressing a 4"-isovalyl transferase gene (*ist*) of a carbomycin-producing strain *(Streptomyces thermotolerans)* in a spiramycin-producing strain *(Streptomyces spiramyceticus* F21) by utilizing a synthetic biology technology. The carrimycin is a multi-component antibiotic and takes 4"-isovalerylspiramycin III, II and I as major components, wherein the components III, II and I are sequentially named by 3-position OR groups, that is, propionyl, acetyl and hydroxyl. A structural formula of the carrimycin is as follows:

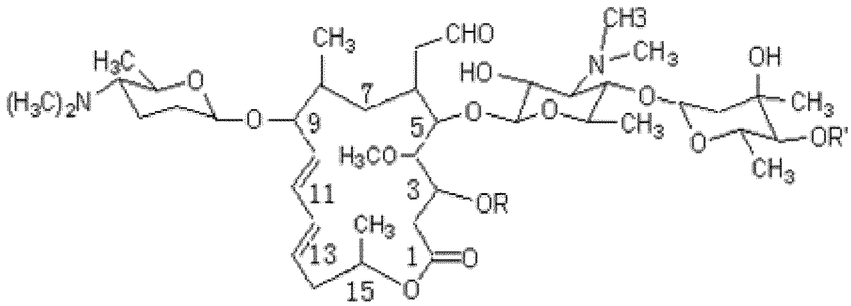

Structural formula of the carrimycin

| | |
|---|---|
| Isovalerylspiramycin III: R=$COCH_2CH_3$ | R' =$COCH_2CH(CH_3)_2$ |
| Isovalerylspiramycin II: R=$COCH_3$ | R' =$COCH_2CH(CH_3)_2$ |
| Isovalerylspiramycin I: R=H | R' =$COCH_2CH(CH_3)_2$ |

Carrimycin serving as a category of new drugs has entered a new drug approval phase; and the specified content of the component III shall not be less than 30% in the quality standard.

A series of regulatory genes exist in *Streptomyces,* and biosynthesis of secondary metabolites may be increased by activating or inhibiting transcription of the regulatory genes, thereby increasing the yield of the metabolites (Bekker V et al. Bioengineered. 2014, 5(5): 293-299). Leucine-responsive regulatory protein (Lrp) is a category of regulatory transcription factors, and activates or inhibits transcriptional activities of the regulatory genes so as to regulate physiological metabolisms of many microbes in combination with regulatory gene promoter sequences (Peeters E et al. Archaea. 2010, Article ID 750457; Unoarumhi Y et al BMC Evol Biol. 2016 16(1): 111), such as metabolism of branched chain amino acids, synthesis of flagella, inhibition of virulence factors, polyamine homeostasis, assimilation of methanol and other processes (Haney SA et al. J Bacteriol. 1992, 174(1): 108-115; Braaten BA et al. Proc Natl Acad Sci U S A. 1992, 89(10): 4250-4; Baek CH et al J Bacteriol. 2009, 191(4): 1278-92; Ishii Y et al. J Biosci Bioeng. 2018, 125(1): 67-75; Gonzalez JE et al. Metab Eng. 2018 45:67-74). However, there are few research reports on functions and effects of the gene Lrps in antibiotic biosynthesis. Only Liu et al. (Liu J. et al. Appl Microbiol Biotechnol. 2017, 101(14):5773-5783) discovered a Lrp/AsnC regulatory factor SCO3361 from the Lrp protein family in *Streptomyces coelicolor;* and the lack of the coding gene of SCO3361 led to the ignificant decrease of the yield of actinorhodin, meanwhile, formation of aerial mycelia and spores is inhibited in a solid medium. The Lrp/AsnC regulatory factor SCO3361 is considered as a multi-effect regulatory factor for regulating secondary metabolism and morphological development (Liu J et al. Metab Eng.

2017, 39:29-37). Moreover, a gene SACE_Lrp is discovered from an erythromycin producing strain (*Saccharopolyspora erythraea*); and due to the lack of the gene, the fermentation yield of erythromycin can be increased (Liu J et al. Metab Eng. 2017, 39: 29-37). There is no report on study of increasing the yield and proportion of the antibiotic components by modifying the gene *lrp.*

In view of this, the present disclosure is specially provided.

## SUMMARY

Technical problem solved in the present disclosure is to overcome defects in the prior art and the present disclosure provide a spiramycin-producing strain, a carrimycin-producing strain, construction method therefor, use thereofand a method for increasing the product yield. In the present disclosure, by inactivating a gene Lrp, the yield of spiramycin is increased; and the yield and proportion of a major component 4"-isovalerylspiramycin III of carrimycin, a derivative of the spiramycin, are significantly increased.

To solve the above technical problems, a basic concept of technical solutions adopted in the present disclosure is as follows:

In the laboratory, a gene *orf_5591-lrp* is discovered from a spiramycin-producing strain (S. *spiramyceticus* 1941) by virtue of bioinformatics analysis, and has homology of 93%/34% to transcriptional regulation factors of an Lrp/AsnC family of *S. flavidovirens*. By analyzing a promoter region of positive regulatory genes *orf42 (srm40* homologous gene) and *orf23 (srm22* homologous gene) in *S. spiramyceticus* and a sequence between a positive regulatory gene *acyB2* and a gene *ist* in *S. thermotolerans,* it is discovered that they all contain a feature binding sequence YAGHAWATTWTDCTR of Lrp (H=non-G, W=A or T, D=non-C). A purpose of the present disclosure is to increase the yield of the spiramycin and the yield and proportion of the major component 4"-isovalerylspiramycin III of carrimycin, the derivative of the spiramycin, by modifying the gene *lrp.*

A first purpose of the present disclosure is to provide a spiramycin-producing strain. A gene Lrp in the spiramycin-producing strain is inactivated; and the strain is deposited as a preservation number of CGMCC No. 16056.

A second purpose of the present disclosure is to provide a carrimycin-producing strain. A gene Lrp in the carrimycin-producing strain is inactivated; and the strain is deposited as a preservation number of CGMCC No. 16055.

According to a further solution, in the above spiramycin-producing strain or the carrimycin-producing strain, a nucleotide sequence of the gene Lrp is shown as Seq.1; and preferably, an amino acid sequence of a protein Lrp is shown as Seq.2.

A third purpose of the present disclosure is to provide a method for constructing a spiramycin-producing strain and/or a carrimycin-producing strain. The method includes the following steps:

(1) constructing a recombinant vector for inactivating a gene Lrp; and introducing the recombinant vector into a spiramycin-producing original strain and/or a carrimycin-producing original strain; and
(2) screening to obtain a recombinant strain with the inactivated gene Lrp.

According to a further solution, by taking a genome of the spiramycin-producing original strain or the carrimycin-producing original strain as a template and taking complete or partial knockout of the gene Lrp as a principle, primers are designed at suitable sites; a left arm gene segment and a right arm gene segment are subjected to PCR amplification; a recombinant vector is constructed by cloning the left arm gene segment and the right arm gene segment to a knockout vector; and the recombinant vector is introduced into the spiramycin-producing original strain and/or the carrimycin-producing original strain, and then the recombinant bacterium with the inactivated gene Lrp is obtained by virtue of resistance and passage screening.

A fourth purpose of the present disclosure is to provide use of the spiramycin-producing strain in increasing the yield of spiramycin. The gene Lrp in the spiramycin-producing strain is inactivated.

A fifth purpose of the present disclosure is to provide use of the carrimycin-producing strain in increasing the yield of a component III of carrimycin. The gene Lrp in the carrimycin-producing strain is inactivated.

A sixth purpose of the present disclosure is to provide a method for increasing a yield of a component III of spiramycin and/or a component III of carrimycin. The method includes the following steps:

(1) modifying a gene Lrp on a chromosome of a spiramycin-producing original strain or a carrimycin-producing original strain to inactivate the gene Lrp; and
(2) performing fermentation production by using a modified spiramycin-producing strain or a modified carrimycin-producing strain in step (1).

According to a preferred solution, after the gene Lrp in the spiramycin-producing original strain or the carrimycin-producing original strain is inactivated, the yield of the component III of spiramycin and/or the component III of carrimycin

is increased by increasing expressions of spiramycin-associated biosynthetic pathway related genes, alanine biosynthetic pathway related genes and acyl coenzyme A metabolic pathway related genes.

According to a further solution, the modified spiramycin-producing strain or the modified carrimycin-producing strain is subjected to seed culture and fermentation culture, and fermentation liquor is subjected to extraction, and the fermentation product obtained is detected and analyzed by HPLC.

According to a further solution, the above gene Lrp inactivation includes insertion, mutation, partial knockout, or complete knockout.

With the adoption of the above technical solutions, compared with the prior art, the present disclosure has beneficial effects as follows:

In the present disclosure, the gene Lrp on the chromosome of the spiramycin-producing strain or the carrimycin-producing strain is modified so as to inactivate the gene Lrp, thereby facilitating expressions of the spiramycin-associated biosynthetic pathway related genes. The high expressions of the alanine biosynthetic pathway related genes increases the supply of a precursor, that is, propionyl, of the spiramycin component III; and the increase of the expressions of related genes of the acyl coenzyme A metabolic pathway promotes the synthesis of fatty acid chains in spiramycin macrolide ring formation. Therefore, the yield of the spiramycin is increased; and the yield and proportion of the major component 4"-isovalerylspiramycin III of carrimycin, the derivative of the spiramycin, are also greatly increased.

Specific implementation modes of the present disclosure will be further described below in detail in combination with drawings.

## BRIEF DESCRIPTION OF THE DRAWINGS

Accompanying drawings serving as one part of the present disclosure are used for providing a further understanding for the present disclosure. Illustrative embodiments and descriptions thereof in the present disclosure are used for explaining the present disclosure, rather than forming an inappropriate limitation to the present disclosure. Apparently, the accompanying drawings in the description below are merely some of the embodiments of the present disclosure, based on these accompanying drawings, other drawings may be obtained by those of ordinary skill in the art without any creative effort. In the accompanying drawings:

Fig. 1 is a possible 3D configuration of a protein Lrp;
Fig. 2 is a SDS-PAGE electrophoretogram of a purified protein Lrp;
wherein, 1-mycelia before induction; 2-mycelia after induction; 3-precipitate after induction; 4-supernatant after induction; 5-protein after purification; M-Protein Marker;
Fig. 3 shows EMSA analysis of binding of a protein Lrp to promoter regions of *Porf42*, *Porf23* and *Pist-acyB2*;
wherein, C1-P*orf42*-Lrp compound; C2-P*orf23*-Lrp compound; C3-P*ist-acyB2*-Lrp compound; N-non-specific DNA control (a sequence on a pQE9 vector);
Fig. 4 shows PCR verification of aΔlrp-SP mutant strain;
wherein, *1-S. spiramyceticus* 1941 original strain; 2-Δ*lrp*-SP mutant strain; M-DNA marker;
Fig. 5 is a detection result diagram of HPLC detection;

> wherein, A: HPLC detection of content of a fermentation product, that is, spiramycin SP, of a Δ*lrp*-SP mutant strain; B: HPLC detection of a fermentation product, that is, carrimycin BT, of a Δ*lrp*-BT mutant strain;

Fig. 6 is qPCR analysis of the Δ*lrp*-SP mutant strain;

> wherein, horizontal coordinate: a gene comp4553_c0_seq2 is related to a macrolide antibiotic biosynthetic pathway; a gene comp9112_c0_seq1 is related to an alanine biosynthetic pathway; and a gene comp8771_c0_seq1 is related to an acyl coenzyme A metabolic pathway;
> vertical coordinate: a quantitative analysis relative value of gene expressions.

It should be indicated that, these drawings and texts are not intended to limit the concept scope of the present disclosure in any manner, but to explain the concept of the present disclosure for those skilled in the art by referring to specific embodiments.

## DETAILED DESCRIPTION

To make the objectives, technical solutions and advantages of the embodiments of the present disclosure clearer, a clear and complete description of the technical solutions in the embodiments will be given below, in combination with the accompanying drawings in the embodiments of the present disclosure. The embodiments below are used for

illustrating the present disclosure, rather than limiting the scope of the present disclosure.

Embodiment 1

Expression and purification of protein Lrp in *Escherichia coli*

By utilizing primers *lrp*-F and *lrp*-R (shown as Table 1), PCR was conducted by taking total DNA of a *S. spiramyceticus* 1941 original strain as a template (Tang Li, et al. Chinese Journal of Biotechnology 1991, 124-131) so as to obtain gene Lrp segment; the gene Lrp segment was cloned to a vector pQE9 (purchased from Shanghai Junrui Biotechnology Co., Ltd.); a N terminal of the gene Lrp was fused with a label His6; and then a recombinant plasmid pQE9-*lrp* was constructed. Then, E. *coli* BL21 (DE3) (Beijing TransGen Biotech Co., Ltd.) was transformed by the recombinant plasmid; a transformant was cultured in an LB medium; induced expression was conducted at 0.5 mM IPTG at 16°C for 8-10 h; the protein Lrp fused with the label His6 was purified with a column $Ni^{2+}$-NTA (QIAGEN). A possible 3D configuration of the protein Lrp is as shown in Fig. 1. The purified protein is detected by SDS-PAGE. Results are as shown in Fig. 2.

Table 1 Primers used in the experiment

| Sequence | Sequence (5'-3') | Purpose |
|---|---|---|
| *lrp*-F<br>*lrp*-R | CGGGATCCACCGGATATTCACCGGACGCC<br>CCCAAGCTTGCGGCTGAGCGCCCTGCGCG | Expression of gene lrp in E. coli |
| *lrp*-sgRNA-F<br>*lrp*-sgRNA-R | GGACTAGTCATGTCCGCGAGCGCCGTGAGT TTTAGAGCTAGAAAT<br>GCTCTAGACTCAAAAAAAGCACCGACTCGG | Guide sequence of Cas9 cleavage sites |
| *lrp*-AF<br>*lrp*-AR<br>*lrp*-109LysR | GCTCTAGAAGCTGGCGCACGAGTTCTT<br>CCCAAGCTTGTCGCTGGAGGCGGAGTT<br>GCACGGCTCCGCGGTGCGGCACCACATGAC | Construction of *lrp* truncated plasmid |
| *lrp*-109LysF | GGGGATCACAGCACGAAGCA TCCCCGTCATGTGGTGCCGCACCGCGGAGC CGTGC | pKC-*lrp*LR |
| *lrp*-CF | GTGGGGGCCTGGGGAGTG | Verification primers of |
| *lrp*-CR | ACCGCAGCGTGGGCATCG | Δ*lrp*-SP mutant strain |
| *lrp*-NF<br>*lrp*-NR | AGGCTCCGCCCCCCTGACGA<br>GAGAAAGCGCCACGCTTCCCG | Amplification in EMSA analysis Control DNA in pQE9 sequence |
| *pIA-F*<br>*pIA*-R | GGGTGACCTCGCGGCGGACG<br>TATGACCCTCACAAGCCGCT | Amplification of promoter region sequence between ist and acyB2 genes |
| *orf* 23-F<br>*orf* 23-R | AGTCGCCGGTAGCTTATCC<br>TACGAATACAATGACAGGTTA | Amplification of *orf* 23 promoter region |
| *orf* 42-F<br>*orf* 42-R | GGCAGGTAGGCCATTTGGG<br>TCGTCATTCCTGCGAGTGG | Amplification of *orf42* promoter region |
| *ist-F*<br>*ist-R* | TCGGTGTGGGACGGACTGCT<br>CGCGCCGGGGTCGTAGTGGT | Identification of *ist* gene in Δ*lrp*-SP and Δ*lrp*-BT strains |
| 4553-F<br>4553-R | GGGCGTGTCGGGGTCGGGCG<br>CGGACGAACTGTGGGACCTG | Amplification of gene comp4553_c0_seq2 during qRT-PCR analysis |
| 9112-F<br>9112-R | CCGAGAGGCTGCCGCCTCAG<br>GACCTGGTGATCCTCATCA | Amplification of gene comp9112_c0_seq1 in qRT-PCR |

(continued)

| Sequence | Sequence (5'-3') | Purpose |
|---|---|---|
| 8771-F<br>8771-R | GCGCAGCCGGTCGACGTACC<br>TCCACCAGCCGGAGGAGGCC | Amplification of gene comp8771_c0_seq1 during qRT-PCR analysis |
| 16S-RNA-F<br>16S-RNA-R | GGTAGAGCTTGTTGACGCAGAG<br>ATGAGGGCGAGGACAGCGATGC | Amplification of 16S RNA serving as internal reference during qRT-PCR analysis |

Embodiment 2

By virtue of gel shift or electrophoretic mobility shift assay *(EMSA),* binding of a protein *Lrp* and promoter regions of regulatory genes such as *ist-acyB2, orf42 (srm40* homologous gene) and *orf23 (srm22* homologous gene) was detected.

PCR was conducted by utilizing corresponding primers (as shown in Table 1); and the sequence segment between *ist-acyB2* genes and the sequence segments of promoter regions of *orf42* and *orf23* were respectively obtained. 0.4 ng of DNA reacted with the protein *Lrp* of different concentrations (such as 0.4, 1.0 and 2.0 ng); the reaction was carried out in a 20 μL solution containing 10 mM of Tris-Cl (pH 7.5), 50 mM of NaCl, 1 mM of EDTA and 4 mM of DTT and 5% (v/v) glycerin. A partial sequence on a 0.1 ng/μL of pQE9 vector was taken as control DNA; 300 μg/mL of acetylated bovine serum albumin served as control. The reaction was carried out at a room temperature for 20 min; 10 μL of the post-reaction solution of each group was taken for electrophoresis in 5% polyacrylamide gel Tris-boric acid-EDTA buffer (having a pH value of 8.7); staining was conducted with a GelRed solution (BioTium) for 20 min; water washing was conducted with water twice; and scanning was performed by a gel imaging system (Tanon).

The results show that, the protein *Lrp* may be respectively bound to the promoter regions of the *ist-acyB2, orf42* and *orf*23 (Fig. 3), which indicates that the protein *Lrp* is a category of transcriptional regulation factors. It is speculated that, the protein *Lrp* can regulate the positive regulatory genes such as *acyB2, orf42* and *orf23* in biosynthesis of spiramycin (BT) or carrimycin (SP), thereby affecting biosynthetic pathways of the BT or SP.

Embodiment 3

Construction of gene *lrp* deleted spiramycin-producing strain Δ*lrp*-SP mutant strain

To study effects of *Lrp* on spiramycin regulatory genes and influences of the *Lrp* on biosynthesis of the spiramycin, the gene Lrp was deleted by 327 bp by utilizing a CRISPR-cas9 *Streptomyces* gene editing system (Huang et al. Acta Biochim Biophys Sin (Shanghai), 2015, 47(4): 231-243) and an overlap PCR method. By utilizing designed primers such as *lrp*-AF and *lrp*-109LysR, and primers such as *lrp*-109LysF and *lrp*-AR, 1.3 kb and 1.0 kb of DNA fragments on left and right arms of the gene Lrp were respectively amplified; the two fragments were ligated by virtue of the overlap PCR; the fragments were cloned to a vector pKCcas9dO through restriction enzyme cutting sites such as *XbaI* and *Hind* III (Huang, et al., Acta Biochim Biophys Sin (Shanghai), 2015, 47(4): 231-243), thereby obtaining a recombinant vector pKC-*lrp*LR.

The constructed recombinant vector was transformed into *S. spiramyceticus* 1941; and a mutant strain was selected through passage and resistance screening. Through the PCR and sequencing verification, the results are as shown in Fig. 4. It is proved that the gene Lrp is successfully truncated; and thus the Δ*lrp*-SP mutant strain is successfully constructed.

The Δ*lrp*-SP mutant strain constructed in the present disclosure is a gene Lrp deleted spiramycin-producing strain, has a classification name of *Streptomyces spiramyceticus,* was preserved in China General Microbiological Culture Collection Center on July 04, 2018, has a preservation address of #3, Yard 1, West Beichen road, Chaoyang District, Beijing, and has a preservation number of CGMCC No. 16056.

The method adopted in the present embodiment is merely for describing contents of the present disclosure, rather than limiting the present disclosure. To implement the present disclosure, two pairs of primers may be designed at suitable sites by those skilled in the art by general molecular biology techniques by taking the genome of the spiramycin-producing strain and the carrimycin-producing strain as a template and taking a damaged gene Lrp structure as a principle; gene segments on left and right arms were subjected to PCR amplification and then cloned to a vector; then

a recombinant vector was constructed; and producing bacteria were introduced in a homologous gene recombination form, thereby obtaining the gene Lrp damaged mutant strain.

Embodiment 4

Construction of gene Lrp deleted carrimycin-producing strain Δ*lrp*-BT strain

To synthesize carrimycin from the Δ*lrp*-SP mutant strain, a plasmid pSET-*ermE*p-ist* (Zhang Jiahu, et al., Chinese Journal of Biotechnology, 2014, 30(9): 1390-1400) was transformed into the Δ*lrp*-SP mutant strain; and an original strain *S. spiramyceticus* 1941 was transformed by pSET-*ermE*p-ist,* thereby obtaining the gene Lrp deleted carrimycin-producing strain Δ*lrp*-BT and a control strain *S. spiramyceticus* 1941-BT. The Δ*lrp*-BT mutant strain constructed in the present disclosure is the gene Lrp deleted carrimycin-producing strain, has a classification name of *Streptomyces spiramyceticus,* was preserved in China General Microbiological Culture Collection Center on July 04, 2018, has a preservation address of #3, Yard 1, West Beichen road, Chaoyang District, Beijing, and has a preservation number of CGMCC No. 16055.

In the present disclosure, the gene Lrp can be directly damaged in the carrimycin-producing strain (ZL97104440.6, ZL021487711.5) by using the universal method in Embodiment 3, thereby obtaining the Δ*lrp*-BT strain.

Embodiment 5:

Yield and component detection of fermentation products of Δ*lrp*-SP and Δ*lrp*-BT mutant strains

A 30 ml/250 ml shake flask was inoculated with spores of the mutant strains and original strain control strains; seeds were cultured in a TSB (Kieser T et al. 2000, Practical Streptomyces genetics, P412) culture medium at 28°C for 48 h; fermentation culture was conducted at 30°C for 6 d. An appropriate amount of fermentation fluid was taken and centrifuged at a rate of 10000 rpm for 10 min; a supernatant was quantitatively taken and extracted with ethyl acetate. The extracted precipitate was dissolved with equivalent acetonitrile; the solution was filtered by a 0.22μm organic phase filter membrane; and then HPLC detection was conducted.

Detection conditions were as follows: an Agilent high performance liquid chromatograph (1200Series) was used; a chromatographic column was YMC-Triart C18, 5 μm, 4.6 mm×250 mm; a mobile phase was 100% of acetonitrile and 10 mM of ammonium acetate (having a pH of 8.0) according to a ratio of 1:1; a flow rate was 1 mL/min; and a detection wavelength was 232 nm. The component content of the SP and the BT was quantitatively analyzed by a peak area.

The Δ*lrp*-SP and Δ*lrρ*-BT mutant strains and the original strains *S. spiramyceticus* 1941 and 1941-BT were fermented and extracted and then subjected to HPLC detection. The results are as shown in Fig. 5; and peak area data of liquid chromatography is as shown in Table 2.

Table 2 HPLC detection data of components of the SP and BT

| Strain | SP I or ISP I (%) | SP II or ISP II (%) | SP III or ISP III (%) | SP or ISP (%) |
|---|---|---|---|---|
| *S. spiramyceticus* 1941 | 12.0±0.4 | 39.9±2.2 | 24.4±3.6 | 76.3±1.6 |
| Δ*lrp*-SP | 11.1±0.3 | 37.4±3.2 | 33.0±6.1 | 81.5±3.1 |
| 1941-BT | ND | 13.5±1.5 | 4.2±0.2 | 17.7±1.7 |
| Δ*lrρ*-BT | ND | 10.4±1.5 | 8.4±2.5 | 18.9±1.6 |

It can be seen from the results that, the SP yield in the Δ*lrp*-SP mutant strain is increased from 76.3% of the original strain to 81.5%; while the proportion of the SP III is greatly increased compared with that of the *S. spiramyceticus* 1941 and increased from 24.4% of the original strain to 33.0%. The proportion of the SP III is increased by 1.37 times. The total yield of the ISP in the Δ*lrp*-BT mutant strain is higher than that of the *S. spiramyceticus* 1941-BT mutant strain, and is increased from 17.7% of the original strain to 18.9%; while the proportion of the ISP III is significantly increased, and increased from 4.2% of the original strain to 8.4%. The yield of the component ISP III is doubled. The results of the two mutant strains are consistent, which forcefully proves that damage of a structural domain of the protein Lrp can increase the yield of the component III.

Embodiment 6

Content detection of amino acids in the Δ*lrp*-SP mutant strain

To detect influences of damage of the gene *lrp* on amino acid metabolism, amino acid detection was conducted on the Δ*lrp*-SP mutant strain and the *S. spiramyceticus* 1941 control strain in the present embodiment. A detection method was as follows:

(1) preparation of a standard curve: an amino acid mixed standard solution served as an external standard.
(2) 100 mg of microbes was added into a 5 mL tube; 5 steel balls were added; the tube with the microbes was frozen in liquid nitrogen for 5 min. Then the tube was put into a high-throughput tissue lyser at 70 Hz for 1 min. 1600 μL of (1:50) diluted hydrochloric acid was added, and vortex oscillation was performed for 30 s. 400 μL of 50 ppm norvaline (50 μg/mL) was added to serve as an internal standard, and vortex oscillation was performed for 30 s. The solution was treated in an ultrasonic machine at room temperature for 30 min. 750 μL of chloroform was added, and vortex oscillation was performed for 1 min. the solution was centrifuged at 12000 rpm and 4°C for 10 min. 500 μL of the supernatant was taken and transferred into a new 1.5 mL centrifuge tube; the sample was concentrated by a vacuum centrifugal concentrator. 90 μL of a methoxy solution (15 mg/mL, dissolved in pyridine) was added to the centrifuge tube, and vortex oscillation was performed for 30 s; a reaction was carried out at 37°C for 2 h; finally 60 μL of a BSTFA reagent (containing 1% of trimethylchlorosilane) was added; a reaction was conducted at 37°C for 90 min. The reaction solution was centrifuged at 12000 rpm at 4°C for 10 min; and the supernatant was added into a detection bottle, wherein detection equipment was an Agilent 7890A/5975C gas chromatography and mass spectrometer. and
(3) chromatographic conditions for amino acid detection were as follows: a chromatographic column was an HP-5MS capillary column (5% phenyl methyl silox: 30 m×250 μm i.d.,0.25-μm; agilent J&W scientific, Folsom, CA); split sampling was conducted; a sample size was 1 μL; a split ratio was 20:1. An injection port temperature was 250 °C; an ion source temperature was 230 °C; an interface temperature was 250 °C; a quadrupole rod temperature was 150 °C. An initial programmed temperature was 70 °C; the temperature was maintained for 2 min, and raised to 300 °C at a rate of 10 °C/min and then maintained for 5 min. The total operation time was 30 min. Carrier gas was helium. A flow rate of the carrier gas was 1 mL/min; and solvent delay time was 4 min. MS conditions were as follows: electron bombardment ion (EI) source; full scan and SIM scanning modes; and electron energy of 70 eV

Content data results of major amino acids associated with spiramycin biosynthesis are listed into Table 3. It is discovered that, the content of intracellular branched chain amino acids (Val, Leu and Ile) in the Δ*lrp*-SP mutant strain is significantly decreased compared with that of the original strain *S. spiramyceticus* 1941.

Table 3 Comparison of content of intracellular amino acids in the Δ*lrp*-SP mutant strain and the *S. spiramyceticus* 1941

| Sample name (μg/g) | Δ*lrp*-SP mutant strain | *S. spiramyceticus* 1941 |
|---|---|---|
| Val | 32.0±4.1 | 65.1±5.6 |
| Leu | 37.8±9.5 | 62.6±5.1 |
| Ile | 13.5±3.2 | 25.1±2.5 |

Embodiment 7

qPCR detection for expressions of spiramycin biosynthesis related genes in Δ*lrp*-SP mutant strain

Expression situations of a gene comp4553_c0_seq2 related to spiramycin-associated biosynthetic pathways, a gene comp9112_c0_seq1 related to alanine biosynthetic pathways and a gene comp8771_c0_seq1 related to acyl coenzyme A metabolic pathways were verified by utilizing qPCR. An instrument Light Cycler 96 (Roche) was used for detection during real-time quantitative PCR. The Δ*lrp*-SP mutant strain and *S. spiramyceticus* 1941 control strain grew in the TSB fluid medium for 60 h; mycelia were respectively centrifuged and collected and then washed with P-Buffer twice. Total RNA was extracted by a bacterial total RNA extraction kit (purchased from Tiangen Biotechnology Co., Ltd.); residual DNA in the total RNA was removed with DNAaseI (Beijing TransGen Biotechnology Co., Ltd.). The total RNA was subjected to inverse transcription by a StarScript II First-strand cDNA Synthesis Kit (GenStar Biotechnology) so as to synthesize cDNA; and then gene expressions were quantitatively detected by utilizing a qPCR detection kit (Faststart Essential DNA Green Master, Roche). Reaction conditions were as follows:

1) pre-denaturation at 96°C for 300s;

2) melting at 96°C×10 s; annealing at 60°C×15 s; extension at 72°C×10 s; × 45 cycles;

The annealing temperature is moderately regulated according to different Tm values of the primers

Melting conditions:

| | |
|---|---|
| 95°C | 10s |
| 65°C | 60s |
| 97°C | 1s |

The result is as shown in Fig. 6. Compared with that in the control strain, an expression quantity of the gene comp8771_c0_seq1 in the Δ*lrp*-SP mutant strain is increased by 5.72 times; an expression quantity of the gene comp9112_c0_seq1 is increased by 11.47 times; and an expression quantity of the gene comp4553_c0_seq2 is increased by 6.34 times.

It was confirmed by qPCR that the deletion of the gene Lrp promoted the expressions of genes related to the spiramycin-associated biosynthetic pathway. The high expressions of genes related to the alanine biosynthetic pathway increased the supply of propionyl, the precursor of the spiramycin component III. And the increase of the expressions of genes related to the acyl coenzyme A metabolic pathway promoted the synthesis of fatty acid chains in spiramycin macrolide ring formation. Therefore, it can be proved theoretically that, the gene Lrp is related to spiramycin biosynthesis, particularly synthesis of the component III.

The above descriptions are merely preferred embodiments of the present disclosure, rather than a limitation of any form to the present disclosure. Although the present disclosure has been disclosed above in the preferred embodiments, the embodiments are not used for defining the present disclosure. Changes or modifications made by those skilled in the art by virtue of the above technical contents without departing from the scope of the technical solutions of the present disclosure are equivalent embodiments with equivalent changes. However, simple improvements, equivalent changes and modifications made to the above embodiments according to the technical essence of the present disclosure without departing from the contents of the technical solutions of the present disclosure shall belong to the scope of the solutions in the present disclosure.

SEQUENCE LISTING

<110> Shenyang Fuyang Pharmaceutical Technology Co., Ltd.

<120> Spiramycin-producing strain, carrimycin-producing strain, construction method therefor, use thereof and method for increasing product yield thereof

<160> 2

<170> PatentIn version 3.5

<210> 1
<211> 447
<212> DNA
<213> Spiramycin-producing strain 1941(S. spiramyceticus 1941)

<400> 1
```
atgaccggat attcaccgga cgccaccgac tggcgcatcc tggaagccct gcaaagcgaa       60

ggccgggcca gtttcaccga gctggcacgc accgtctcca tgtccgcgag cgccgtgacg      120

gagcgggtac ggcggctcga agaggccggt gtgatctcgg gctacatggc tgtcgtcgag      180

caggaccggc tcggaatgcc catcctggcg ttcgtacggc tgcgctatcc gcacggcaac      240

tacaagccct tccacgacct gctggacgtg acgcccgaag ttctggaggc gcatcacgtc      300

accggtgacg actgcttcgt gctgaaggtc gcggcccgct cgatgaagca cctcgaagag      360

gtgtcgggcc ggatcggctc gctgggctcg gtcaccacga gcgtcgtcta ctcctccccg      420

ctgccgcgca gggcgctcag ccgctga                                           447
```

<210> 2
<211> 148
<212> PRT
<213> Spiramycin-producing strain 1941(S. spiramyceticus 1941)

<400> 2

```
Met Thr Gly Tyr Ser Pro Asp Ala Thr Asp Trp Arg Ile Leu Glu Ala
1               5                   10                  15

Leu Gln Ser Glu Gly Arg Ala Ser Phe Thr Glu Leu Ala Arg Thr Val
            20                  25                  30

Ser Met Ser Ala Ser Ala Val Thr Glu Arg Val Arg Arg Leu Glu Glu
        35                  40                  45

Ala Gly Val Ile Ser Gly Tyr Met Ala Val Val Glu Gln Asp Arg Leu
    50                  55                  60

Gly Met Pro Ile Leu Ala Phe Val Arg Leu Arg Tyr Pro His Gly Asn
65                  70                  75                  80

Tyr Lys Pro Phe His Asp Leu Leu Asp Val Thr Pro Glu Val Leu Glu
```

```
                85                  90                  95

Ala His His Val Thr Gly Asp Asp Cys Phe Val Leu Lys Val Ala Ala
            100                 105                 110

Arg Ser Met Lys His Leu Glu Glu Val Ser Gly Arg Ile Gly Ser Leu
        115                 120                 125

Gly Ser Val Thr Thr Ser Val Val Tyr Ser Ser Pro Leu Pro Arg Arg
    130                 135                 140

Ala Leu Ser Arg
145
```

## Claims

1. A spiramycin-producing strain, wherein a gene Lrp in the spiramycin-producing strain is inactivated, and the strain is deposited as a preservation number of CGMCC No. 16056.

2. A carrimycin-producing strain, wherein a gene Lrp in the strain is inactivated, and the strain is deposited as a preservation number of CGMCC No. 16055.

3. The spiramycin-producing strain according to claim 1, or the carrimycin-producing strain according to claim 2, wherein an amino acid sequence of a protein Lrp is shown as Seq.2; and
preferably, a nucleotide sequence of the gene Lrp is shown as Seq.1.

4. A method for constructing a spiramycin-producing strain and/or a carrimycin-producing strain, comprising:

    (1) constructing a recombinant vector for inactivating a gene Lrp; and introducing the recombinant vector into a spiramycin-producing original strain and/or a carrimycin-producing original strain; and
    (2) screening to obtain a recombinant strain with the inactivated gene Lrp.

5. The method according to claim 4, wherein by taking a genome of the spiramycin-producing original strain and/or the carrimycin-producing original strain as a template and taking complete or partial knockout of the gene Lrp as a principle, primers are designed at suitable sites; a left arm gene segment and a right arm gene segment are subjected to PCR amplification; a recombinant vector is constructed by cloning the left arm gene segment and the right arm gene segment to a knockout vector; and the recombinant vector is introduced into the spiramycin-producing original strain and/or the carrimycin-producing original strain, and then the recombinant strain with the inactivated gene Lrp is obtained by virtue of resistance and passage screening.

6. Use of a spiramycin-producing strain in increasing the yield of spiramycin, wherein a gene Lrp in the spiramycin-producing strain is inactivated.

7. Use of a carrimycin-producing strain in increasing the yield of a component III of carrimycin, wherein a gene Lrp in the carrimycin-producing strain is inactivated.

8. A method for increasing a yield of a component III of spiramycin and/or a component III of carrimycin, comprising:

    (1) modifying a gene Lrp on a chromosome of a spiramycin-producing original strain or a carrimycin-producing original strain to inactivate the gene Lrp; and
    (2) performing fermentation production by using a modified spiramycin-producing strain or a modified carrimycin-producing strain obtained in step (1);

preferably, after the gene Lrp in the spiramycin-producing original strain or the carrimycin-producing original strain

is inactivated, the yield of the component III of the spiramycin and/or the component III of carrimycin is increased by increasing expressions of spiramycin-associated biosynthetic pathway related genes, alanine biosynthetic pathway related genes and acyl coenzyme A metabolic pathway related genes.

**9.** The method according to claim 8, wherein the modified spiramycin-producing strain or the modified carrimycin-producing strain is subjected to seed culture and fermentation culture, and fermentation liquor is subjected to extraction, and the fermentation product obtained is detected and analyzed by HPLC.

**10.** The spiramycin-producing strain according to claim 1, or the carrimycin-producing strain according to claim 2, or the method according to claim 4, or the use according to claim 6 or 7, or the method according to claim 8, wherein the inactivation of the gene Lrp includes insertion, mutation, partial knockout, or complete knockout.

SEQUENCE LISTING

<110> Shenyang Fuyang Pharmaceutical Technology Co., Ltd.

<120> Spiramycin-producing strain, carrimycin-producing strain, construction method therefor, use thereof and method for increasing product yield thereof

<160> 2

<170> Patent In version 3.5

<210> 1

<211> 447

<212> DNA

<213> Spiramycin-producing strain 1941 (*S. spiramyceticus* 1941)

<400> 1

atgaccggat attcaccgga cgccaccgac tggcgcatcc tggaagccct gcaaagcgaa 60

ggccgggcca gtttcaccga gctggcacgc accgtctcca tgtccgcgag cgccgtgacg 120

gagcgggtac ggcggctcga agaggccggt gtgatctcgg gctacatggc tgtcgtcgag 180

caggaccggc tcggaatgcc catcctggcg ttcgtacggc tgcgctatcc gcacggcaac 240

tacaagccct tccacgacct gctggacgtg acgcccgaag ttctggaggc gcatcacgtc 300

accggtgacg actgcttcgt gctgaaggtc gcggcccgct cgatgaagca cctcgaagag 360

gtgtcgggcc ggatcggctc gctgggctcg gtcaccacga gcgtcgtcta ctcctccccg 420

ctgccgcgca gggcgctcag ccgctga 447

<210> 2

<211> 148

<212> PRT

<213> Spiramycin-producing strain 1941 (*S. spiramyceticus* 1941)

<400> 2

Met Thr Gly Tyr Ser Pro Asp Ala Thr Asp Trp Arg Ile Leu Glu Ala

1 5 10 15

Leu Gln Ser Glu Gly Arg Ala Ser Phe Thr Glu Leu Ala Arg Thr Val

20 25 30

Ser Met Ser Ala Ser Ala Val Thr Glu Arg Val Arg Arg Leu Glu Glu

35 40 45

Ala Gly Val Ile Ser Gly Tyr Met Ala Val Val Glu Gln Asp Arg Leu

50 55 60

Gly Met Pro Ile Leu Ala Phe Val Arg Leu Arg Tyr Pro His Gly Asn

65 70 75 80

Tyr Lys Pro Phe His Asp Leu Leu Asp Val Thr Pro Glu Val Leu Glu

85 90 95

Ala His His Val Thr Gly Asp Asp Cys Phe Val Leu Lys Val Ala Ala

100 105 110

Arg Ser Met Lys His Leu Glu Glu Val Ser Gly Arg Ile Gly Ser Leu

115 120 125

Gly Ser Val Thr Thr Ser Val Val Tyr Ser Ser Pro Leu Pro Arg Arg

130 135 140

Ala Leu Ser Arg

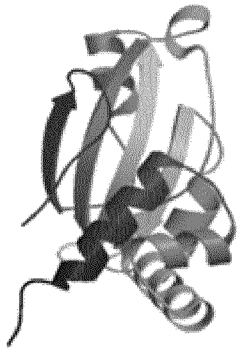

Fig. 1

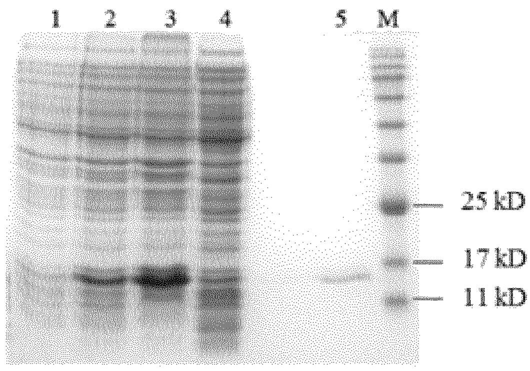


Fig. 2

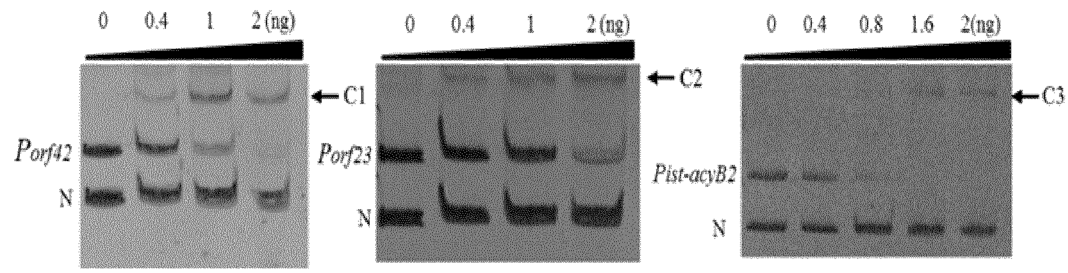


Fig. 3

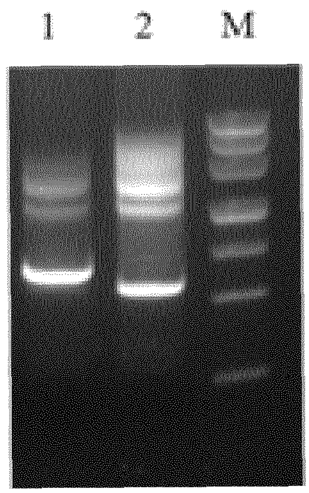
1
2
M

Fig. 4

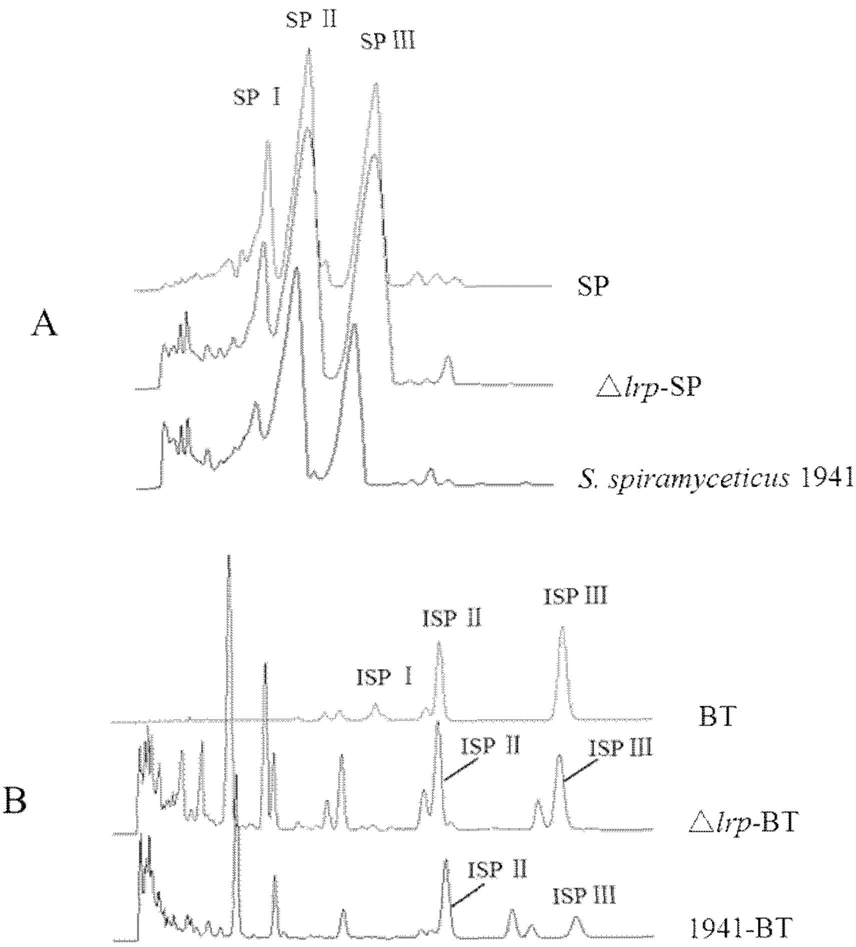
SP II
SP III
SP I
SP
A
△lrp-SP
S. spiramyceticus 1941
ISP III
ISP II
ISP I
BT
ISP II
ISP III
B
△lrp-BT
ISP II
ISP III
1941-BT

Fig. 5

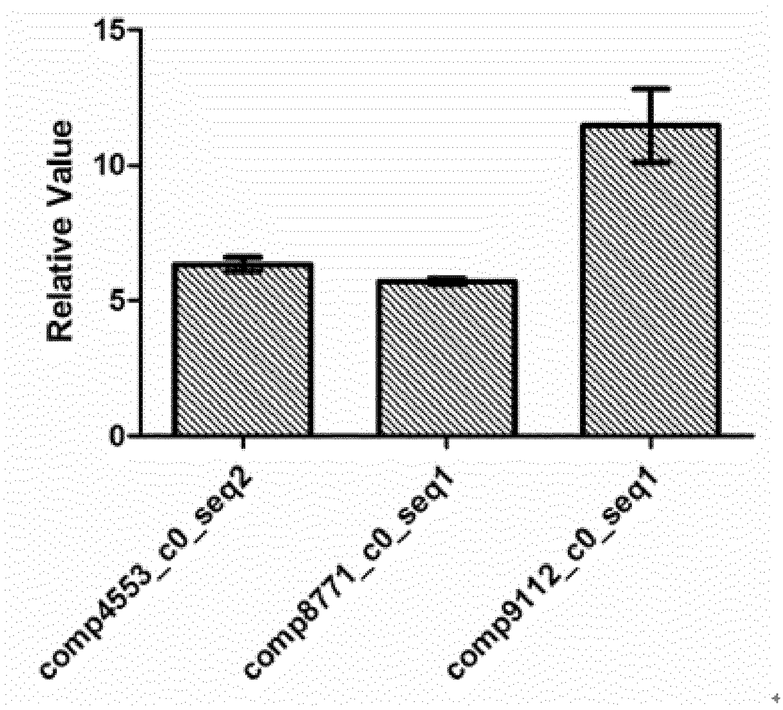
15
10
5
0
Relative Value
comp4553_c0_seq2
comp8771_c0_seq1
comp9112_c0_seq1

Fig. 6

**INTERNATIONAL SEARCH REPORT**

International application No. **PCT/CN2019/124724**

**A. CLASSIFICATION OF SUBJECT MATTER**

C12N 1/21(2006.01)i; C12N 15/31(2006.01)i; C07K 14/36(2006.01)i; C12P 19/62(2006.01)i; C12R 1/465(2006.01)i

According to International Patent Classification (IPC) or to both national classification and IPC

**B. FIELDS SEARCHED**

Minimum documentation searched (classification system followed by classification symbols)

C12N; C07K; C12P; C12R

Documentation searched other than minimum documentation to the extent that such documents are included in the fields searched

Electronic data base consulted during the international search (name of data base and, where practicable, search terms used)

DWPI; CNABS; SIPOABS; CNTXT; CNKI; Google; ISI web of knowledge; 申请人/发明人, 螺旋霉素, 生技霉素, 比特螺旋霉素, 亮氨酸反应蛋白, Lrp, Leucine-responsive regulatory protein, carrimycin, shengjimycin, bitespiramycin, Streptomyces thermotolerans, CGMCC 16056, CGMCC 16055, SEQ ID NO: 2

**C. DOCUMENTS CONSIDERED TO BE RELEVANT**

| Category* | Citation of document, with indication, where appropriate, of the relevant passages | Relevant to claim No. |
|---|---|---|
| PX | LIU, Z.L. et al. "Engineering of leucine-responsive regulatory protein improves spiramycin and bitespiramycin biosynthesis." *MICROBIAL CELL FACTORIES.*, Vol. 18, 19 February 2019 (2019-02-19), abstract | 1-10 |
| Y | 刘静 (LIU, Jing). "亮氨酸应答调控因子Lrp调控抗生素生物合成的分子机制及应用 (Mechanism and Application of Leucine-responsive Regulatory Protein (Lrp) Controlling Antibiotic Biosynthesis)" *中国博士学位论文全文数据库 工程科技I辑 (Chinese Doctoral Dissertations Full-Text Database, Engineering Science & Technology I),* No. 8, 15 August 2017 (2017-08-15), B018-9, abstract | 1-10 |
| Y | 唐莉 等 (TANG, Li et al.). "螺旋霉素聚酮合成酶基因和抗性基因的克隆与表达的研究 (Cloning of Polyketide Synthase Gene and Resistance Gene from Spiramycin Producing Strain)" *生物工程学报 (Chinese Journal of Biotechnology),* Vol. 7, No. 1, 31 December 1991 (1991-12-31), abstract, and page 26, paragraph 1 | 1, 3-6, 8-10 |

☑ Further documents are listed in the continuation of Box C. ☑ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **18 February 2020** | **11 March 2020** |
| Name and mailing address of the ISA/CN<br>**China National Intellectual Property Administration (ISA/CN)**<br>**No. 6, Xitucheng Road, Jimenqiao Haidian District, Beijing 100088**<br>**China** | Authorized officer |
| Facsimile No. **(86-10)62019451** | Telephone No. |

Form PCT/ISA/210 (second sheet) (January 2015)

INTERNATIONAL SEARCH REPORT

International application No.
PCT/CN2019/124724

C. DOCUMENTS CONSIDERED TO BE RELEVANT

| Category* | Citation of document, with indication, where appropriate, of the relevant passages | Relevant to claim No. |
|---|---|---|
| Y | CN 1405299 A (INSTITUTE OF MEDICINAL BIOTECHNOLOGY, CHINESE ACADEMY OF MEDICAL SCIENCES et al.) 26 March 2003 (2003-03-26)<br>description, page 2, paragraphs 3-4 | 2-5, 7-10 |
| A | 钟晶晶 (ZHONG, Jingjing). "碳霉素与必特螺旋霉素生物合成调控的研究 (Regulation of Carbomycin and Bitespiramycin Biosynthesis)"<br>*中国优秀硕士学位论文全文数据库 基础科学辑 (Basic Sciences, China Master's Theses Full-Text Database)*, No. 2, 15 February 2017 (2017-02-15),<br>A006-701, entire document | 1-10 |
| A | PEETERS, E. et al. "The Lrp Family of Transcription Regulators in Archaea."<br>*ARCHAEA.*, Vol. 2010, 31 December 2010 (2010-12-31),<br>Article ID 750457 | 1-10 |
| A | LIU, J. et al. "Characterization of an Lrp/AsnC family regulator SCO3361, controlling actinorhodin production and morphological development in Streptomyces coelicolor."<br>*APPLIED MICROBIOLOGY AND BIOTECHNOLOGY.*,<br>Vol. 101, 11 June 2017 (2017-06-11),<br>pp. 5773-5783 | 1-10 |
| A | WO 2017114034 A1 (SHENYANG TONGLIAN GROUP CO., LTD.) 06 July 2017 (2017-07-06)<br>entire document | 1-10 |

Form PCT/ISA/210 (second sheet) (January 2015)

## INTERNATIONAL SEARCH REPORT

International application No.
**PCT/CN2019/124724**

**Box No. I Nucleotide and/or amino acid sequence(s) (Continuation of item 1.c of the first sheet)**

1. With regard to any nucleotide and/or amino acid sequence disclosed in the international application, the international search was carried out on the basis of a sequence listing:
   a. ☑ forming part of the international application as filed:
      ☑ in the form of an Annex C/ST.25 text file.
      ☐ on paper or in the form of an image file.
   b. ☐ furnished together with the international application under PCT Rule 13*ter*.1(a) for the purposes of international search only in the form of an Annex C/ST.25 text file.
   c. ☐ furnished subsequent to the international filing date for the purposes of international search only:
      ☐ in the form of an Annex C/ST.25 text file (Rule 13*ter*.1(a)).
      ☐ on paper or in the form of an image file (Rule 13*ter*.1(b) and Administrative Instructions, Section 713).

2. ☐ In addition, in the case that more than one version or copy of a sequence listing has been filed or furnished, the required statements that the information in the subsequent or additional copies is identical to that forming part of the application as filed or does not go beyond the application as filed, as appropriate, were furnished.

3. Additional comments:

Form PCT/ISA/210 (continuation of first sheet) (January 2015)

**INTERNATIONAL SEARCH REPORT**
**Information on patent family members**

International application No.
**PCT/CN2019/124724**

| Patent document cited in search report | | | Publication date (day/month/year) | Patent family member(s) | | | Publication date (day/month/year) |
|---|---|---|---|---|---|---|---|
| CN | 1405299 | A | 26 March 2003 | CN | 1169947 | C | 06 October 2004 |
| WO | 2017114034 | A1 | 06 July 2017 | US | 2019093112 | A1 | 28 March 2019 |
| | | | | KR | 20180093083 | A | 20 August 2018 |
| | | | | CL | 2018001796 | A1 | 28 September 2018 |
| | | | | CA | 3009706 | A1 | 06 July 2017 |
| | | | | PH | 12018501393 | A1 | 27 February 2019 |
| | | | | CN | 105505954 | B | 22 January 2019 |
| | | | | PE | 20190221 | A1 | 13 February 2019 |
| | | | | EP | 3385386 | A4 | 10 October 2018 |
| | | | | CN | 105505954 | A | 20 April 2016 |
| | | | | CO | 2018007130 | A2 | 19 July 2018 |
| | | | | JP | 2019500903 | A | 17 January 2019 |
| | | | | CN | 107868789 | A | 03 April 2018 |
| | | | | ZA | 201804397 | B | 25 September 2019 |
| | | | | EP | 3385386 | A1 | 10 October 2018 |
| | | | | AU | 2016383270 | A1 | 26 July 2018 |
| | | | | BR | 112018013309 | A2 | 11 December 2018 |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Non-patent literature cited in the description


- **BEKKER V et al.** *Bioengineered,* 2014, (5), 293-299
- **PEETERS E et al.** *Archaea,* 2010
- **UNOARUMHI Y et al.** *BMC Evol Biol,* 2016, vol. 16 (1), 111
- **HANEY SA et al.** *J Bacteriol,* 1992, vol. 174 (1), 108-115
- **BRAATEN BA et al.** *Proc Natl Acad Sci U S A.,* 1992, vol. 89 (10), 4250-4
- **BAEK CH et al.** *J Bacteriol,* 2009, vol. 191 (4), 1278-92
- **ISHII Y et al.** *J Biosci Bioeng,* 2018, vol. 125 (1), 67-75
- **GONZALEZ JE et al.** *Metab Eng,* 2018, vol. 45, 67-74
- **LIU J et al.** *Appl Microbiol Biotechnol,* 2017, vol. 101 (14), 5773-5783
- **LIU J et al.** *Metab Eng,* 2017, vol. 39, 29-37
- **TANG LI et al.** *Chinese Journal of Biotechnology,* 1991, 124-131
- **HUANG et al.** *Acta Biochim Biophys Sin (Shanghai),* 2015, vol. 47 (4), 231-243
- **ZHANG JIAHU et al.** *Chinese Journal of Biotechnology,* 2014, vol. 30 (9), 1390-1400